# EUROPEAN PATENT SPECIFICATION

(11) **EP 2 108 373 B1**
(45) Date of publication and mention of the grant of the patent: **17.07.2013**
(21) Application number: 09009373.3
(22) Date of filing: 29.10.2001
(51) Int. Cl.: A61K 38/20, A61P 35/00, A61K 31/541, A61K 31/549

(54) **Treatment of tumor metastases and cancer**
Behandlung von Tumor-Metastasen und Krebs
Traitement de métastases tumorales et de cancers

(30) Priority: 27.10.2000 US 243409 P
(43) Date of publication of application: 14.10.2009
(62) Divisional of application: 01309157.4
(73) Proprietor: ED. GEISTLICH SÖHNE AG FÜR CHEMISCHE INDUSTRIE, 6110 Wolhusen (CH)
(72) Inventor: Redmond, Paul H., Wilton, Cork (IE); Pfirrmann, Rolf W., CH-6353 Weggis (CH)
(74) Representative: Matthews, Derek Peter

(56) References cited:
- EP-A- 1 066 830
- WO-A-92/00743
- DATABASE EMBASE [Online] ELSEVIER SCIENCE PUBLISHERS, AMSTERDAM, NL January 2006 O'BRIEN G C ET AL: 'Co-immunotherapy with interleukin-2 and taurolidine for progressive metastatic melanoma' Database accession no. EMB-2006171607

## Description

The present invention relates to the field of treating tumor metastases and cancer.

Interleukin-2 (IL-2) is an agent which has been suggested for inhibiting tumor cell growth. However, administration of IL-2 to patients presents severe toxicity problems, since IL-2 elicits an extremely strong systemic inflammatory response syndrome (SIRS) reaction in patients. Toxicity of IL-2 is so severe that approximately 70% of patients cannot tolerate treatment.

Additionally, a common problem in patients undergoing cancer treatment is tumor recurrence or metastasis.

Thus, despite the advances in cancer treatment, there remains a significant need in the art for new and improved cancer treatment therapies.

In accordance with the present invention, tumor metastasis is inhibited in a cancer patient by administering to said patient a combination therapy comprising effective amounts of IL-2 and a methylol transfer agent.

It has surprisingly been found that the methylol transfer agent taurolidine reduces or substantially eliminates the severe toxicity and side effects of IL-2 in a combination therapy for inhibiting tumor metastases and treating cancer in patients, while it has unexpectedly been found that the efficacy of IL-2 is actually enhanced by the methylol transfer agent in the combination therapy of the present invention.

IL-2 when used in accordance with the present invention includes natural or recombinant Interleukin-2, or biologically active derivatives or substantial equivalents thereof.

Methylol transfer agents include taurolidine. The compounds taurolidine and taurultam are disclosed in U.S. Patent No. 5,210,083. Other methylol-containing compounds may be found among those identified in PCT Publication No. WO 01/39763. The methylol transfer agents for utilization in accordance with the present invention is taurolidine.

The Preferred embodiments of the invention involve treatment of cancers selected from the group consisting of malignant melanoma and inhibition of tumor metastases thereof.

Effective daily dosage amounts of IL-2 may comprise pharmaceutical dosage units within the range of 1,000,000-100,000,000 units (U) IL-2 per m² body surface area. Dosage amounts of IL-2 also may be found within the range of 100,000-1,000,000 U per kilogram body weight. Dosage amounts of IL-2 further may be found within the range of 0.1-100 micrograms IL-2 per kilogram body weight.

Effective dosage amounts of taurolidine in accordance with the present invention may comprise pharmaceutical dosage units within the range of about 0.1-1,000 mg/kg. Preferred dosages may be in the range of about 10-20 grams taurolidine

Pharmaceutical dosage units of the combined therapy of the present invention may be administered by any suitable route, which include oral, topical or peritoneal administration, e.g., subcutaneously, intraperitoneally, intramuscularly, or intravenously. eg., by infusion or injection.

In preferred embodiments, 250 ml of taurolidine 2% solution is administered by intravenous infusion about 1-6 times per day, more preferably about 2-4 times per day, during a treatment period, concurrently with administration of about 10,000,000-40,000,000 units m² IL-2 by intravenous infusion per day during the treatment period.

The present invention also is directed to a combination of IL-2 and a methylol transfer agent, in effective amounts for simultaneous, separate or sequential use for inhibiting tumor metastasis in a cancer patient. The invention also is directed to pharmaceutical combinations including pharmaceutical dosage units comprising effective amounts of Interleukin-2 and a methylol transfer agent for inhibiting tumor metastasis in a cancer patient, as well as to pharmaceutical compositions comprising such combinations.

The invention is further illustrated by the following examples.

### Example 1

A 63 year old patient diagnosed with metastatic malignant melanoma was treated as follows.

| | |
|---|---|
| Presentation | Right supra-clavicular mass. Originally had nodular melanoma excised from right elbow, and had high-dose interferon post-operatively. Required axillary clearance for a mass in right axilla eight months later. Further staging was clear at that time. Presented one year later with a fixed inoperable mass in right supra--clavicular area. |
| Treatment | IL-2 and Taurolidine |
| Regimen | Interleukin-2 |
| | Day 1: 18 million units/m²IL-2 infusion over 6 hours |
| | Day 2: 18 million units/m² IL-2 infusion over 12 hours |
| | Day 3: 18 million units/m² IL-2 infusion over 24 hours |
| | Days 4-7: 18 million units/m² IL-2 infusion over 78 hours |
| | Taurolidine |
| | Taurolidine 2% 250 ml infusion over twelve hours, daily during IL-2 administration |
| | Completed five courses of the above |

After one year, the patient is alive and well, with no evidence of disease on imaging.

### Example 2

A 50 year old patient diagnosed with metastatic renal cell carcinoma was treated as follows.

| | |
|---|---|
| Presentation | Haemoptysis - 2° to pulmonary metastases. Noted to have hepatic metastases, in addition to a large mass in the left kidney. |
| Treatment | IL-2 and Taurolidine |
| Regimen | Interleukin-2 |
| | Day 1: 18 million units/m² IL-2 infusion over 6 hours |
| | Day 2: 18 million units/m² IL-2 infusion over 12 hours |
| | Day 3: 18 million units/m² IL-2 infusion over 24 hours |
| | Days 4-7: 18 million units/m² IL-2 infusion over 78 hours |
| | Taurolidine |
| | Taurolidine 2% 250 ml infusion over two hours, twice daily during IL-2 administration |
| | Completed five courses of the above |

### Further treatment Left radical nephrectomy

After five years, the patient is alive and well, with no evidence of disease on imaging.

### Example 3

A male patient who had recurrent nodular melanoma after interferon treatment was subsequently treated with Interleukin-2 and Taurolidine as follows:

| | |
|---|---|
| Presentation | Recurrence of nodular melanoma lesion in right shoulder. |
| Treatment | IL-2 and Taurolidine |
| Regimen | Interleukin-2 |
| | Day 1: 36 million units/m² IL-2 infusion over 6 hours |
| | Day 2: 36 million units/m² IL-2 infusion over 12 hours |
| | Day 3: 36 million units/m² IL-2 infusion over 24 hours |
| | Days 4-7: 36 million units/m² IL-2 infusion over 78 hours |
| | Taurolidine |
| | Taurolidine 2% 250 ml infusion over twelve hours, sequentially with IL-2 administration, during days 1-6 |
| | Undertook five courses -- during second course, treatment was interrupted and stopped at 78 hours, and during the fifth course, treatment was interrupted during day 4. |

Follow-up CT scans indicated a reduction in the size of the lesion, and subsequently indicated no evidence of disease.

## Claims

1. Use of taurolidine and interleukin-2 in the manufacture of a combined preparation for simultaneously, separately or sequentially treating malignant melanoma.

2. Use as claimed in claim 1 wherein said combined preparation is manufactured such that said interleukin-2 and said taurolidine are each present in the same pharmaceutical dosage unit.

3. Use as claimed in claim 1 wherein said combined preparation is manufactured such that said interleukin-2 and said taurolidine are each present as separate pharmaceutical dosage units.

## Patentansprüche

1. Verwendung von Taurolidin und Interleukin-2 bei der Herstellung eines Kombinationspräparats zur simultanen, getrennten oder sequenziellen Behandlung eines malignen Melanoms.

2. Verwendung nach Anspruch 1, wobei das Kombinationspräparat dergestalt hergestellt wird, dass das Interleukin-2 und das Taurolidin jeweils in der gleichen pharmazeutischen Dosierungseinheit vorliegen.

3. Verwendung nach Anspruch 1, wobei das Kombinationspräparat dergestalt hergestellt wird, dass das Interleukin-2 und das Taurolidin jeweils als getrennte pharmazeutische Dosierungseinheiten vorliegen.

## Revendications

1. Utilisation de taurolidine et d'interleukine 2 dans la fabrication d'une préparation combinée pour traiter simultanément, séparément ou séquentiellement un mélanome malin.

2. Utilisation selon la revendication 1, dans laquelle ladite préparation combinée est fabriquée de sorte que ladite interleukine 2 et ladite taurolidine sont présentes chacune dans la même unité de dosage pharmaceutique.

3. Utilisation selon la revendication 1, dans laquelle ladite préparation combinée est fabriquée de sorte que ladite interleukine 2 et ladite taurolidine sont présentes chacune en tant qu'unités de dosage pharmaceutique distinctes.
